Europäisches Patentamt

European Patent Office

Office européen des brevets

(11) Publication number: **0 498 137 A1**

## EUROPEAN PATENT APPLICATION

(21) Application number: **91610007.6**

(22) Date of filing: **06.02.91**

(51) Int. Cl.⁵: **C12N 15/56**, C12N 9/24

(43) Date of publication of application:
**12.08.92 Bulletin 92/33**

(84) Designated Contracting States:
**AT BE CH DE DK ES FR GB GR IT LI LU NL SE**

(71) Applicant: **NOVO NORDISK A/S**
**Novo Allé**
**DK-2880 Bagsvaerd(DK)**

(72) Inventor: **Dörreich, Kurt**
**Im Proli 20**
**W-7889 Grenzach-Wyhlen(DE)**
Inventor: **Dalboge, Hendrik**
**Ligustervaenget 63**
**DK-2830 Virum(DK)**
Inventor: **Mikkelsen, Jan Moller**
**Snerlevej 20**
**DK-2820 Gentofte(DK)**
Inventor: **Mischler, Marcel**
**Waldeck 95**
**CH-4204 Himmelried(CH)**
Inventor: **Christensen, Flemming Mark**
**Peter Ochs-Strasse 33**
**CH-4059 Basle(CH)**

(74) Representative: **Bach, Niels et al**
**c/o Novo Nordisk A/S Patent Dept. Novo Allé**
**DK-2880 Bagsvaerd(DK)**

(54) **Novel expression systems.**

(57) A partial amino acid sequence of a $\beta$-1,4-galactanase obtainable by means of *Aspergillus aculeatus* is described, and also a corresponding recombinant DNA sequence, vector and transformed host. Use of the $\beta$-1,4-galactanase or a pectinase preparation enriched with the $\beta$-1,4-galactanase for degradation or modification of plant cell walls is described.

The invention relates to genetic engineering and provides a partial amino acid sequence, preferably an N-terminal amino acid sequence of a $\beta$-1,4-galactanase. $\beta$-1,4-galactanases (EC no. 3.2.1.89) is a group of carbohydrases which degrade galactanes. The authorized enzyme name is 1,4-$\beta$-D-galactan galactohydrolase, but the short term $\beta$-1,4-galactanase is used in this specification with claims. Reference can be made to R.F.H. Dekker and G.N. Richards, "Hemicellulases, their Occurence, Purification, Properties and Mode of Action" in R.S. Tipson and D. Horton, Advances in Carbohydrate Chemistry and Biochemistry, Academic Press 32, 277-352 (1976), R.F.H. Dekker, "The Hemicellulase Group of Enzymes", in J.M.V. Blanchard and J.R. Mitchell, Polysaccharides in Food, Butterworths, 93-108 (1979), and A.G.J. Voragen, F. Geerst and W. Pilnik "Hemicellulases in Enzymatic Fruit Processing", in P. Depuy, Use of Enzymes in Food Technology, Technique et Documentation Lavoisier, 497-502 (1982). Galactanes are found in connection with many gums, agar, and fruit pectins, and they are components of cell walls in e.g. algal, mosses, and fruits.

The above indicated partial amino acid sequence can be used for construction of DNA probes which can be used for screening a genomic library from organisms expressing such enzyme, or a cDNA library, thereby obtaining DNA sequences, which can be used either for an overproduction of $\beta$-1,4-galactanase, if inserted in the microorganism species, from which the parent DNA molecule originated, or for production of $\beta$-1,4-galactanase without accompanying closely related enzymes, if inserted in a host microorganism, which in its not-transformed condition does not produce any enzymes closely related to $\beta$-1,4-galactanase.

Thus, the purpose of the invention is the provision of means and methods for production of $\beta$-1,4-galactanase in better yield and higher purity than hitherto possible, and of a use of $\beta$-1,4-galactanase for degradation of plant cell wall tissue, more efficient than hitherto possible. Also it is the purpose of the invention to provide novel products, wherein the proportion of the $\beta$-1,4-galactanase is either increased or decreased in relation to the proportion in the original product.

The recombinant DNA sequence obtainable according to the invention comprises a DNA sequence coding for a polypeptide having $\beta$-1,4-galactanase activity, or a DNA sequence having substantial sequence homology to such $\beta$-1,4-galactanase coding sequence.

In the following it will be explained in detail how the recombinant DNA sequence according to the invention can be produced.

The strain *Aspergillus aculeatus* DSM CBS 101.43 as a gene donor was fermented in a pilot plant scale in the following way.

An agar substrate with the following composition was prepared in a Fernbach flask:

| Peptone Difco | 6 g |
|---|---|
| Aminolin Ortana | 4 g |
| Glucose | 1 g |
| Yeast extract Difco | 3 g |
| Meat extract Difco | 1.5 g |
| $KH_2PO_4$ Merck | 20 g |
| Malt extract Evers | 20 g |
| Ion exchanged $H_2O$ | ad 1000 ml |

pH was adjusted to between 5.30 and 5.35. Then 40 g of Agar Difco was added, and the mixture was autoclaved for 20 minutes at 120°C (the substrate is named E-agar).

The strain CBS 101.43 was cultivated on an E-agar slant (37°C). The spores from the slant were suspended in sterilized skim-milk, and the suspension was lyophilized in vials. The contents of one lyophilized vial was transferred to the Fernbach flask. The flask was then incubated for 13 days at 30°C.

A substrate with the following composition was prepared in a 500 litre seed fermenter:

| $CaCO_3$ | 1.2kg |
|---|---|
| Glucose | 7.2 kg |
| Rofec (corn steep liquor dry matter) | 3.6 kg |
| Soy bean oil | 1.2kg |

Tap water was added to a total volume of around 240 litres. pH was adjusted to around 5.5 before addition of $CaCO_3$. The substrate was sterilized in the seed fermenter for 1 hour at 121°C. Final volume before inoculation was around 300 litres.

The Fernbach flask spore suspension was transferred to the seed fermenter. Seed fermentation conditions were:

Fermenter type: Conventional aerated and agitated fermenter with a height/diameter ratio of around 2.3.

| | |
|---|---|
| Agitation: | 300 rpm (two turbine impellers) |
| Aeration: | 300 normal litre air per minute |
| Temperature: | 30 to 31°C |
| Pressure: | 0.5 ato |
| Time: | around 28 hours |

Around 28 hours after inoculation 150 litres was transferred from the seed fermenter to the main fermenter.

A substrate with the following composition was prepared in a 2500 litre main fermenter:

| | |
|---|---|
| Toasted soy meal | 90 kg |
| $KH_2PO_4$ | 20 kg |
| Pluronic® | 150 ml |

Tap water was added to a total volume of around 900 litres. The toasted soy meal was suspended in water. pH was adjusted to 8.0 with NaOH, and the temperature was raised to 50°C. Thereafter around 925 Anson units of Alcalase® 0.6 L was added to the suspension. The mixture was held for 4 hours at 50°C and pH = 8.0 ($Na_2CO_3$ addition) with no aeration, zero ato and 100 rpm agitation. Thereafter the remaining substrate components were added and pH was adjusted to around 6.0 with phosphoric acid. The substrate was sterilized in the main fermenter for 1½ hours at 123°C. Final volume before inoculation was around 1080 litres.

Then 150 litres of seed culture was added.

Fermentation conditions were:

Fermenter type: Conventional aerated and agitated fermenter with a height/diameter ratio of around 2.7.

| | |
|---|---|
| Agitation: | 250 rpm (two turbine impellers) |
| Aeration: | 1200 normal litre air per minute |
| Temperature: | 30°C |
| Pressure: | 0.5 ato |
| Time: | around 151 hours |

From 24 fermentation hours to around 116 fermentation hours pectin solution was added aseptically to the main fermenter at a constant rate of around 8 litres per hour. The pectin solution with the following composition was prepared in a 500 litre dosing tank:

| | |
|---|---|
| Pectin genu[*] | 22 kg |
| Phosphoric acid, conc. | 6 kg |
| Pluronic® | 50 ml |

[*] Genu pectin (citrus type NF from the Copenhagen pectin factory Ltd.)

Tap water was added to a total volume of around 325 litres. The substrate was sterilized in the dosing tank for 1 hour at 121°C. Final volume before start of dosage was around 360 litres. When this portion ran out, another similar portion was made. Total volume of pectin solution for one fermentation was around 725 litres.

After around 151 fermentation hours the fermentation process was stopped. The around 1850 litres of culture broth were cooled to around 5°C and the enzymes were recovered according to the following method.

The culture broth was drum filtered on a vacuum drum filter (Dorr Oliver), which was precoated with Hyflo Super-Cell diatomaceous earth (filter aid). The filtrate was concentrated by evaporation to around 15%

3

of the volume of the culture broth. The concentrate was filtered on a Seitz filter sheet (type supra 100) with 0.25% Hyflo Super-Cell as a filter aid (in the following table referred to as filtration 1). The filtrate was precipitated with 561 g of $(NH_4)_2SO_4$/l at a pH of 5.5, and 4% Hyflo Super-Cell diatomaceous earth is added as a filter aid. The precipitate and the filter aid are separated by filtration on a frame filter. The filter cake is dissolved in water, and insoluble parts are separated by filtration on a frame filter. The filtrate is check filtered on a Seitz filter sheet (type supra 100) with 0.25% Hyflo Super-Cell as a filter aid (in the following table referred to as filtration II). The filtrate is diafiltered on an ultrafiltration apparatus. After diafiltration the liquid is concentrated to a dry matter content of 12.7% (in the following table referred to as dry matter content in concentrate).

A facultative base treatment for partial removal of the protease activity can be carried out at this stage. In case the base treatment is used it is carried out at a pH of 9.2 for 1 hours, whereafter the pH value is adjusted to 5.0.

Now the liquid is check filtered and filtered for the purpose of germ reduction and the filtrate is freeze-dried on a freeze-drying equipment from Stokes.

Four fermentations were carried out in the manner indicated below, whereby the strain used for the fermentation, the use of the facultative base treatment and other parameters were varied, as indicated in the following table.

4

| Microorganism | Base treatment | | Concentration (%) filter aid in connection with | | | Dry matter content in concentrate | Remarks |
|---|---|---|---|---|---|---|---|
| | used | not used | filtration I | the precipitation | filtration II | | |
| CBS 101.43 | | X | 0.5 | 5 | 0.2 | 28 | |
| ATCC 20236 | | X | 2.0 | 4 | 0.4 | 7.5 | |
| IFO 4408 | | X | 1.0 | 5 | 0.25 | 12.4 | *) |
| CBS 101.43 | X | | 0.25 | 4 | 0.25 | 12.7 | |

*) After germ reducing filtration the filtrate is concentrated by evaporation in a ratio of 1:2.3. A minor part of the concentrated filtrate was spray-dried, and the remaining part was freeze-dried. This crude preparation is an *Aspergillus aculeatus* enzyme preparation which in the following for the sake of brevity will be identified as A.a.e.p.

EP 0 498 137 A1

As shown in Table 1, the $\beta$-1,4-galactanase can be isolated from the above indicated *Aspergillus aculeatus* enzyme preparation broth in the following manner.

ad 1:

Concentration and buffer exchange in order to prepare for step 2, removal of small particles and about 50% of the colour

ad 2:

HIC is hydrophobic interaction chromatography. The colorless $\beta$-1,4-galactanase fraction is pooled from step 0. 8-0.2M $(NH_4)_2SO_4$.

ad 3:

a - Dilution in order to reduce the salt concentration in the sample b - Buffer exchange in order to prepare for step 4

ad 4:

IEG is ion exchange chromatography. The $\beta$-1,4-galactanase fraction is pooled from step 0.25-0.5M NaCl

ad 5:

Buffer adaption in order to prepare for step 6.

ad 6:

The active galactanase fraction was pooled in the range of 0.4M $(NH_4)_2SO_4$. This fraction shows with IEF (isoelectric focusing) one band. On SDS-PAGE (sodium dodecyl sulfate polyacrylamide gel electrophoresis)/silver 3 protein bands were detected, where the main fraction amounts to more than 80% of the protein in the test. The same pictures appeared with/without DTT (dithio threitol). Immuno-blotting generated the same picture as SDS-PAGE/silver.

All efforts to separate these 3 peaks failed. With HPLC RP-chromatography (RP is short for reverse phase) these 3 peaks were found again. Further investigations on the end terminals indicated that the sample is a pure enzyme fraction, and that the accompanying bands are artefacts originating from the sample preparation and working conditions.

## Table 1

### β-1,4-galactanase purification

> **Aspergillus aculeatus enzyme broth**

> **UF - DIALYSIS**
> 1: Filtron Minisette, filter area 1400 $cm^2$, membrane NMWL 10.000
> $H_2O$ + 1M $(NH_4)_2SO_4$, pH 5.0, 5 x volume

> **2: HIC: PHENYL SEPHAROSE CL 4B; FIG. 2**
> (column 7.5 x 20 cm, flow 0.8 ml/minute)
> eluent = $H_2O$, pH 5.0, decreasing $(NH_4)_2SO_4$-step gradient:
> 1M - 0.8M - 0.2M - 0.0M, final washing with 0.1M NaOH

> **3a : ANALYTICAL**
> sample dilution with $H_2O$
> 1: 1.5, pH-adjustment 6.5

> **3b : PREPARATIVE**
> UF-dialysis; 50mM TRIS
> pH 6.5, 5 x volume

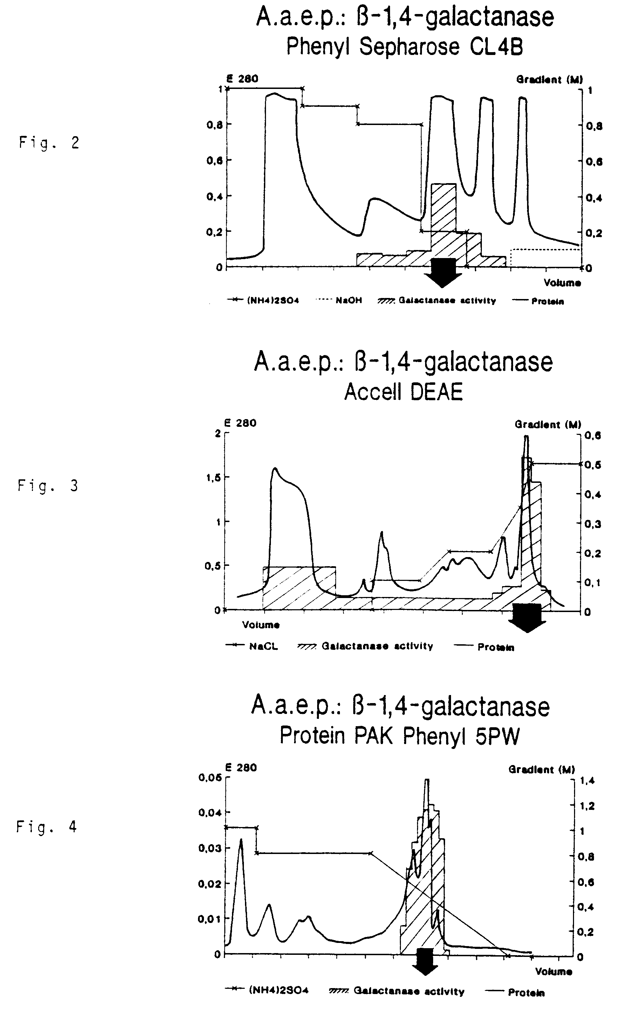

> **4 : IEC: WATERS ACCELL DEAE; FIG. 3**
> (column 2.6 x 30 cm, flow 5.0 ml/minute)
> 50mM TRIS, pH 6.5, increasing NaCl-gradient:
> 0.0M-step-0.1M-step-0.2M-step.0.25M-linear-0.4M-step-0.5M

> **5 : SAMPLE PREPARATION**
> DEAE-galactanase-pool, addition of solid $(NH_4)_2SO_4$ to
> 1M concentration and pH-adjustment to 5.0

> **6 : HIC: WATERS PROTEIN PAK - PHENYL 5PW; FIG. 4**
> (column 0.8 x 7.5 cm, flow 0.6 ml/minute)
> eluent = $H_2O$, pH 5.0, decreasing $(NH_4)_2SO_4$-gradient:
> 1M-step-0.8M-linear-0.0m

> **ENDO-β-1,4-GALACTANASE**

The below indicated Table 2 shows how the enrichment factor increases as the purification proceeds.

Table 2

| Step | Procedure | Protein (mg) | Enzyme activity (U-units) | Specific activity (U/mg) | Enzyme yield (%) | Enrichment factor |
|---|---|---|---|---|---|---|
| Initial | crude enzyme | 2.842 | 539.000 | 190 | 100 | 1.0 |
| 2 | Phenyl Sepharose CL4B | 847 | 440.000 | 519 | 82 | 2.7 |
| 4 | Accell DEAE | 31 | 330.000 | 10.645 | 61 | 56.1 |
| 6 | Protein PAK Phenyl 5PW | 8 | 250.000 | 31.250 | 46 | 164.8 |

The U unit indicated in Table 2 is the $\beta$-1,4-galactanase activity unit, which is defined as follows: 1 unit is the amount of enzyme which at 30°C and in 1 minute releases 1 $\mu$mole of galactose.

Now the N-terminal amino acid sequence is determined:

```
1                    5                         10
Ala-Leu-Thr-Tyr-Arg-Gly-Ala-Asp-Ile-Ser-Xaa-Leu-Leu-Leu

15                   20                        25
Leu-Glu-Asp-Glu-Gly-Tyr-Ser-Tyr-Lys-Asn-Leu-Asn-Gly-Gln-
```

Xaa represents an amino acid, which has not been determined. The sample was not pyridethylated, therefore it was not possible to identify Cys. Position 11 can either be a Cys or a post-translational modified amino acid as a glucosylated Ser.

From the N-terminal sequence the purity of the sample is estimated to be more than 90%.

The N-terminal amino acid sequence of galactanase shows no homology with other proteins in the UW-GCG data bank, a publicly available data bank, in relation to which UW is an abbreviation for University of Wisconsin.

The $\beta$-1,4-galactanase is further characterized, as indicated in the following.

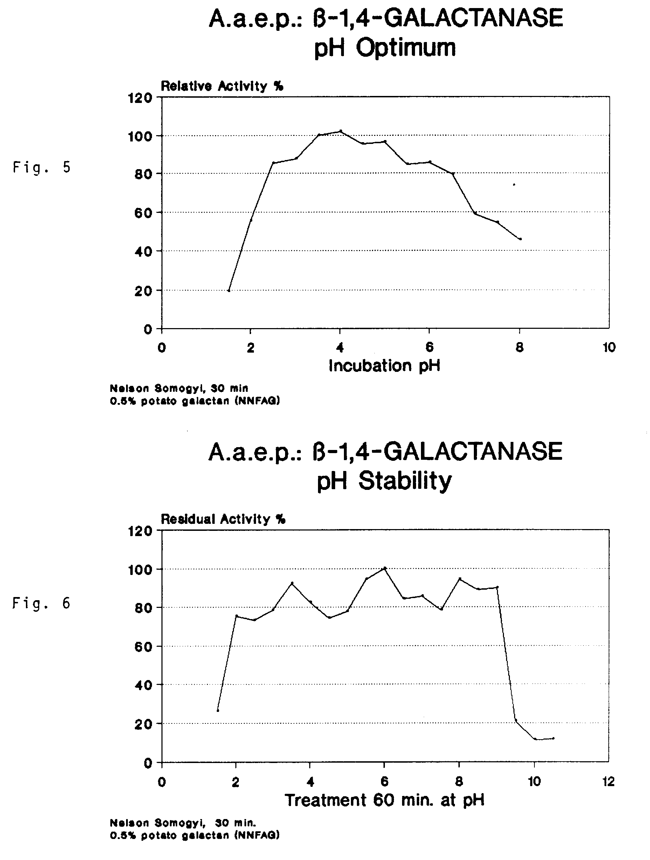

Figs. 5 and 6 show the pH activity and pH stability, respectively, of the $\beta$-1,4-galactanase.

This $\beta$-1,4-galactanase shows a relatively broad pH-spectrum with pH-optimum at pH 3.5 - 4.0

The $\beta$-1,4-galactanase is relatively acid stable. The stability is good between pH 2 and 8, when treated for 1 hour at room temperature.

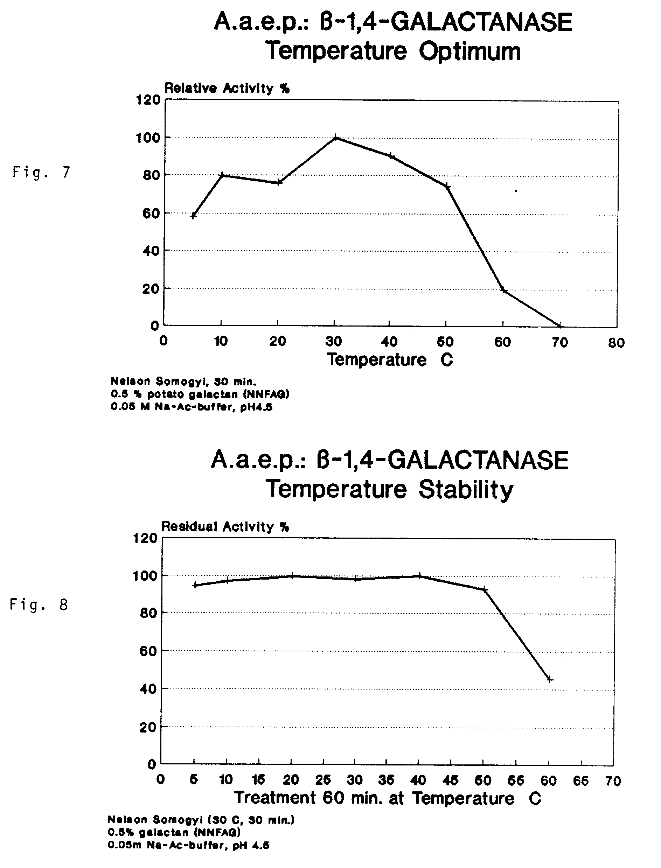

Figs. 7 and 8 show the temperature activity dependency and the temperature stability dependency, respectively, of the $\beta$-1,4-galactanase.

The temperature optimum of the $\beta$-1,4-galactanase is around 30°C, and the temperature activity range is relatively broad. For the fruit juice and wine area the activity in the low temperature range is very remarkable:

around 60% activity at 5°C

around 80% activity at 10°C      In the temperature range of 5 - 55°C the $\beta$-1,4-galactanase activity is not remarkably influenced after a treatment of 1 hour at pH 4.5 ($\geq$ 80% of the initial activity).

Molecular weight:   40.000 Dalton

Isoelectric point:   pH 3.8

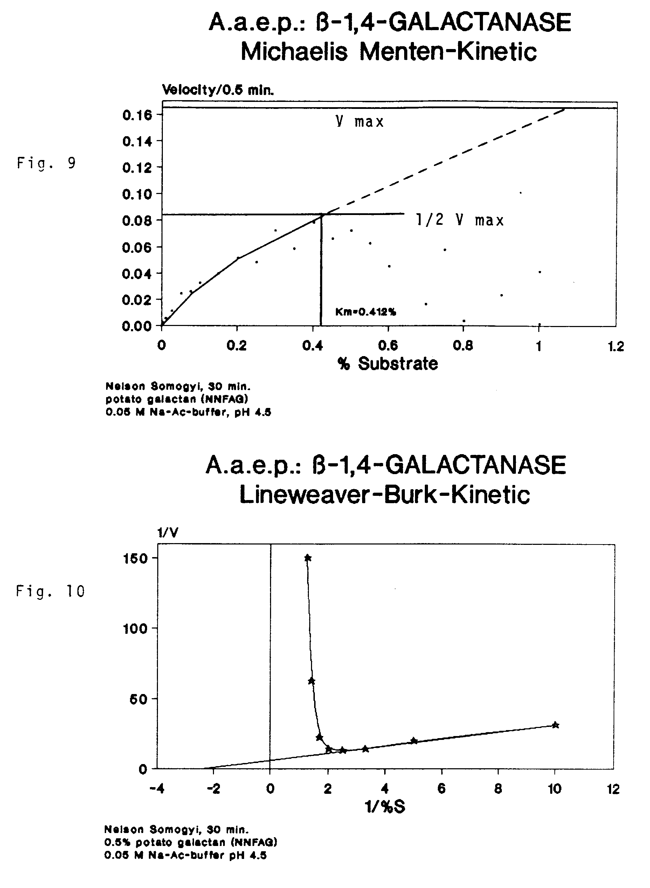

Km-value:   The Michaelis-Menten-Kinetic and the corresponding Lineweaver-Burk-kinetic appears from Figs. 9 and 10, respectively. The resulting Km-value is expressed in %-substrate concentration (not in mole/l, the reason for this being the inhomogenity of the molecular weight distribution of the substrate; therefore it was not possible to calculate an accurate figure for the molecular weight).

The $\beta$-1,4-galactanase is inhibited by substrate concentrations $\geq$ 0.3%. The theoretical and calculated Km-value is

Km = 0.41% NNFAG-potato-galactan

On the basis of the above indicated amino acid sequence a probing process was carried out for the corresponding cDNA. After isolation of the mRNA, the cDNA was synthetized.

A recombinant DNA molecule according to the invention is constructed and identified in the following manner.

Construction of a *A. aculeatus* cDNA library in *E. coli*

Total RNA is extracted from homogenized A. *aculeatus* mycelium, collected at the time for maximum activity of the β-1,4-galactanase, using methods as described by Boel et a. (EMBO J., 3: 1097-1102, 1984) and Chirgwin et al. (Biochemistry (Wash), 18: 5294-5299, 1979). Poly(A)-containing RNA is obtained by two cycles of affinity chromatography on oligo(dT)-cellulose as described by Aviv and Leder (PNAS, USA 69:1408-1412, 1972). cDNA is synthesized with the use of a cDNA synthesis kit from Invitrogen according to the manufacturer's description.

Identification of *A. aculeatus* β-1,4-galactanase specific cDNA recombinants by use of synthetic oligodeoxyribonucleotides

A mixture of synthetic oligodeoxyribonucleotides corresponding to a part of the determined amino acid sequence is synthesized on an Applied Biosystems. Inc. DNA synthesizer and purified by polyacylamide gel electrophoresis. Approximately 150.000 E. *coli* recombinants from the *A. aculeatus* cDNA library is transferred to Whatman 540 paper filters. The colonies are lysed and immobilized as described by Gergen et al. (Nucleic Acids Res 7, 2115-2135, 1979). The filters are hybridized with the $^{32}$P-labelled β-1,4-galactanase specific oligo mixture as described by Boel et al. (EMBO J., 3, 1097-1102, 1984). Hybridization and washing of the filters are done at a temperature 10°C below the calculated Tm, followed by autoradiography for 24 hours with an intensifier screen. Following autoradiography, the filters are washed at increasing temperatures followed by autoradiography for 24 hours with an intensifier screen. Miniprep plasmid DNA is isolated from hybridizing colonies by standard procedures (Birnboim and Doly Nucleic Acids Res 7, 1513-1523, 1979), and the DNA sequence of the cDNA insert is established by the Sanger dideoxy procedure. The β-1,4-galactanase cDNA fragment is exised from the vector by cleavage with HindIII/XbaI (or other appropriate enzymes) and is purified by agarose gel electrophoresis electroeluted and made ready for ligation reactions. The cDNA fragment is ligated to HindIII/XbaI digested pHD414 to generate pHD β-1,4-galactanase in which the cDNA is under transcriptional control of the TAKA promotor from *Aspergillus oryzae* and the AMG terminator from *Aspergillus niger*.

Identification of *A. aculeatus* β-1,4-galactanase specific cDNA recombinants using immunological screening procedures

The cDNA library was split in 50 pools each containing approximately 3000 different cDNA clones. DNA was isolated from the pools and transformed into an appropriate yeast strain. Approximately 20.000 yeast clones (10 plates) were obtained from each of the original pools, in order to ensure that all clones were represented in the yeast library. The yeast clones were replica plated onto minimal agar plates containing galactose. Nitrocellulose filters were placed on top of the yeast colonies followed by incubation of the plates for 2 days at 30°C. The nitrocellulose filters were peeled off and incubated with a monospecific antibody raised against the β-1,4-galactanase, using standard immunological procedures. Positive clones were purified and rescreened twice using the same antibody preparations. DNA was isolated from the positive yeast clones, and transformed into E. *coli* MC1061 in order to get higher quantities of DNA. DNA was isolated and analyzed by use of restriction enzymes. The DNA sequence was determined as previously described. Simultaneously the cDNA was exised from the yeast/E. *coli* vector using HindIII/XbaI, purified on gel and inserted into the *Aspergillus* expression vector pHD414 as described in this specification.

Construction of *Aspergillus* expression vector pHD414

The vector pHD414 is a derivative of the plasmid p775 (described in European patent application no. 87103806.3). In contrast to p775, pHD414 has a string of unique restriction sites between the promotor and the terminator.

The plasmid was constructed by removal of an approximately 200 bp long fragment (containing undesirable restriction sites) at the 3' end of the terminator, and subsequent removal of an approximately 250 bp long fragment at the 5' end of the promotor, also containing undesirable restriction sites.

The 200 bp region was removed from p775 by cleavage with NarI (positioned in the pUC vector) and XbaI (positioned just 3' to the terminator), subsequent filling in at the generated ends using Klenow DNA polymerase + dNTP, purification of the vector fragment on gel and religation of the vector fragment. The DNA was transformed into E. *coli* MC1061 as described above. 10 colonies (pHD413-1 to -10) were selected and analyzed by restriction enzyme analysis. One of the clones exhibiting the expected band pattern in the restriction enzyme analysis was used in the construction of pHD414.

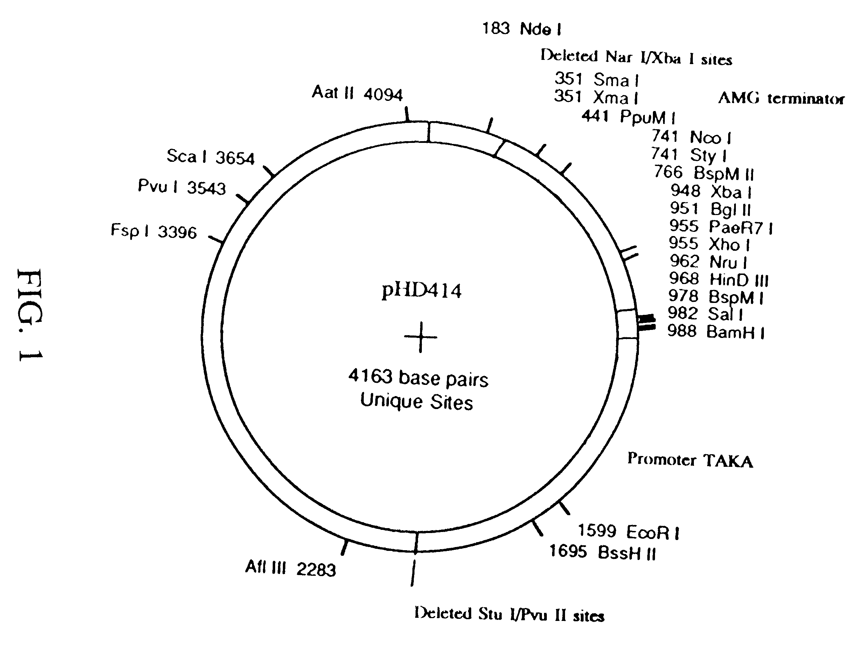

pHD413 was cut with StuI (positioned in the 5' end of the promoter) and PvuII (positioned in the pUC vector) and fractionated on gel. The vector fragment was purified, religated and transformed into *E. coli* MG1061. 12 colonies were selected and analyzed by restriction enzyme analysis. All 12 clones exhibited the expected band pattern. The plasmid pHD414 is shown in Fig. 1.

## Transformation of *Aspergillus oryzae* or *Aspergillus niger* (general procedure)

100 ml of YPD (Sherman et al., Methods in Yeast Genetics, Cold Spring Harbor Laboratory, 1981) is inoculated with spores of *A. oryzae*, *A. niger* or argB mutants hereof and incubated with shaking at 37°C for about 2 days. The mycelium is harvested by filtration through miracloth and washed with 200 ml of 0.6 M $MgSO_4$. The mycelium is suspended in 15 ml of 1.2 M $MgSO_4$. 10 mM $NaH_2PO_4$, pH = 5.8. The suspension is cooled on ice and 1 ml of buffer containing 120 mg of Novozym® 234, batch 1687 is added. After 5 minutes 1 ml of 12 mg/ml BSA (Sigma type H25) is added and incubation with gentle agitation continued for 1.5-2.5 hours at 37°C until a large number of protoplasts is visible in a sample inspected under the microscope.

The suspension is filtered through miracloth, the filtrate transferred to a sterile tube and overlayered with 5 ml of 0.6 M sorbitol, 100 mM Tris-HCl, pH = 7.0. Centrifugation is performed for 15 minutes at 100 g and the protoplasts are collected from the top of the $MgSO_4$ cushion. 2 volumes of STC (1.2 M sorbitol, 10 mM Tris-HCl, pH = 7.5. 10 mM $CaCl_2$) are added to the protoplast suspension and the mixture is centrifugated for 5 minutes at 1000 g. The protoplast pellet is resuspended in 3 ml of STC and repelleted. This is repeated. Finally the protoplasts are resuspended in 0.2-1 ml of STC.

100 $\mu$l of protoplast suspension is mixed with 5-25 $\mu$g of the appropriate DNA in 10 $\mu$l of STC. Protoplasts from the argB strains are mixed with pSal43 DNA (an *A. nidulans* argB gene carrying plasmid) and protoplasts from the $argB^+$ strains are mixed with p3SR2 (an *A. nidulans* amdS gene carrying plasmid). The mixture is left at room temperature for 25 minutes. 0.2 ml of 60% PEG 4000 (BDH 29576). 10 mM $CaCl_2$ and 10 mM Tris-HCl, pH = 7.5 is added and carefully mixed (twice) and finally 0.85 ml of the same solution is added and carefully mixed. The mixture is left at room temperature for 25 minutes, spun at 2500 g for 15 minutes and the pellet is resuspended in 2 ml of 1.2 M sorbitol. After one more sedimentation the protoplasts are spread on the appropriate plates. Protoplasts from the argB strains transformed with pSal43 are spread on minimal plates (Cove Biochem.Biophys.Acta 113 (1966) 51-56) with glucose and urea as carbon and nitrogen sources, respectively, and containing 1.2 M sorbitol for osmotic stabilization. Protoplasts from the argB-strains transformed with p3SR2 are spread on minimal plates (Cove Biochem.Biophys.Acta 113 (1966) 51-56) containing 1.0 M sucrose, pH = 7.0, 10 mM acetamide as nitrogen source and 20 mM CsCl to inhibit background growth. After incubation for 4-7 days at 37°C spores are picked, suspended in sterile water and spread for single colonies. This procedure is repeated and spores of a single colony after the second reisolation is stored as a defined transformant.

Production of $\beta$-1,4-galactanase in high yield with this transformed host:

## Expression of recombinant *A. aculeatus* $\beta$-1,4-galactanase in an *A. oryzae* strain

pHD $\beta$-1,4-galactanase is transformed into *A. oryzae* IFO 4177 by cotransformation with p3SR2 containing the amdS gene from *A. nidulans* as described with a mixture of equal amounts of pHD $\beta$-1,4-galactanase and p3SR2 (approximately 5 $\mu$g of each). Transformants which can use acetamide as sole nitrogen source are reisolated twice. After growth on YPD (Sherman et al. 1981) for three days culture supernatants are analysed by SDS-PAGE. The gels are stained with coomassie brilliant blue R. The best transformants are selected for further studies and grown in a 2 liter Kieler fermentor on 4% soy bean meal and supplied with glucose during growth. The culture is heavily agitated during fermentation. The recombinant product is isolated from the culture broth by removal of the cells by centrifugation, ultrafiltration of the supernatant and freeze drying.

## Expression of $\beta$-1,4-galactanase in an *A. niger* strain

pHD $\beta$-1,4-galactanase is transformed into *A. niger* argB by cotransformation with pSal43 containing the argB gene form *A. nidulans* as described earlier. Protoplast are incubated with equal amounts, approximately 5 $\mu$g of each plasmid. Transformants are selected on minimal plates (Cove Biochem.Biophys.Acta 113 (1966), 55-56) by relief of argenine requirement.

After two reisolations of conidiospores the transformants are cultured for seven days in YPD (Sherman et al., 1981) at 30°C. The culture supernatants are analyzed by SDS-PAGE. Most of the transformants produced $\beta$-1,4-galactanase in their supernatants.

Production of $\beta$-1,4-galactanase using a transformed host other than *Aspergillus* species without significant amounts of accompanying similar enzymes.

## Expression of $\beta$-1,4-galactanase in *S. cerevisiae*

The $\beta$-1,4-galactanase gene is isolated from pHD $\beta$-1,4-galactanase and introduced into the yeast expression vector pYHD5 in which the cDNA is under transcriptional control of the Gal 1-10 promoter and the $\alpha$-factor terminator. A URA3 mutant yeast strain is transformed with the yeast expression plasmid by the LiAc procedure. Transformants are selected on minimal agar plates without uracil. The transformants are replica plated to minimal agar plate without uracil, but supplemented with galactose (in order to induce the promoter) and tested for expression of $\beta$-1,4-galactanase by use of antibodies and by measurement of the enzyme activity.

## Expression of $\beta$-1,4-galactanase in *E. coli*

The $\beta$-1,4-galactanase cDNA is excised from pHD $\beta$-1,4-galactanase using HindIII/XbaI. The fragment is treated with Klenow DNA polymerase and dNTP in order to make blunt ended DNA molecules and purified on gel. The fragment is cloned into the vector pHD282 in the PvuII site (Dalboege et al., Gene, 79, 325-332, 1989). and in a subsequent mutationstep using standard site directed mutagenesis techniques, fused directly in frame to the OmpA signal peptide in pHD282.

The OmpA-$\beta$-1,4-galactanase chimeric gene is transferred to the expression vector pHD 234 as a ClaI/BamHI fragment and transferred into *E. coli* MC1061 (Casadaban and Cohen, J. Mal.Biol., 138, 179-207, 1980) to generate recombinant clones. *E. coli* MC1061 containing the expression plasmid is grown in 1.5 liter MBR reactor equipped with temperature, pH, air-flow rate and agitation controllers. The medium contained 40 mg tryptone/ml (Difco) and 20 mg yeast extract/ml. Production of $\beta$-1,4-galactanase is induced by raising the temperature from 28°C to above 37°C at an $A_{525}$ = 50.

The bacteria samples are analyzed by SDS-PAGE and activity measurements.

The $\beta$-1,4-galactanase according to the invention can be used as a plant cell wall degrading enzyme, thus including the applications shown on page 35 of GB 2115820A.

If the $\beta$-1,4-galactanase according to the invention is used together with the A.a.e.p., a synergistic effect can be demonstrated, especially in regard to TLP (total liquefaction power) on apple mash.

TLP is the total liquefaction activity of an enzyme preparation. This activity is the total effect of all the single activities in this enzyme preparation. The reaction is followed rheologically. The decrease of the total viscosity (= a function of serum + structural viscosity) in a finely milled apple mash is monitored continuously within 2 hours with a rotary viscosimeter.

The enzymes are compared on a MOU-equal basis (Most Units), where per definition the
TLP of Pectinex AP-18 = 0, and the
TLP of Pectinex Ultra SP-L = 100

A more detailed description of the MOU unit is to be found in the brochure "Determination of the pectinase units on apple juice (MOU)", which can be obtained on request from Novo Nordisk Ferment (Schweiz) AG, Neumatt, CH-4243 Dittingen, Switzerland.

Table 3 shows the TLP figures obtained by the galactanase alone and in combination with the A.a.e.p.

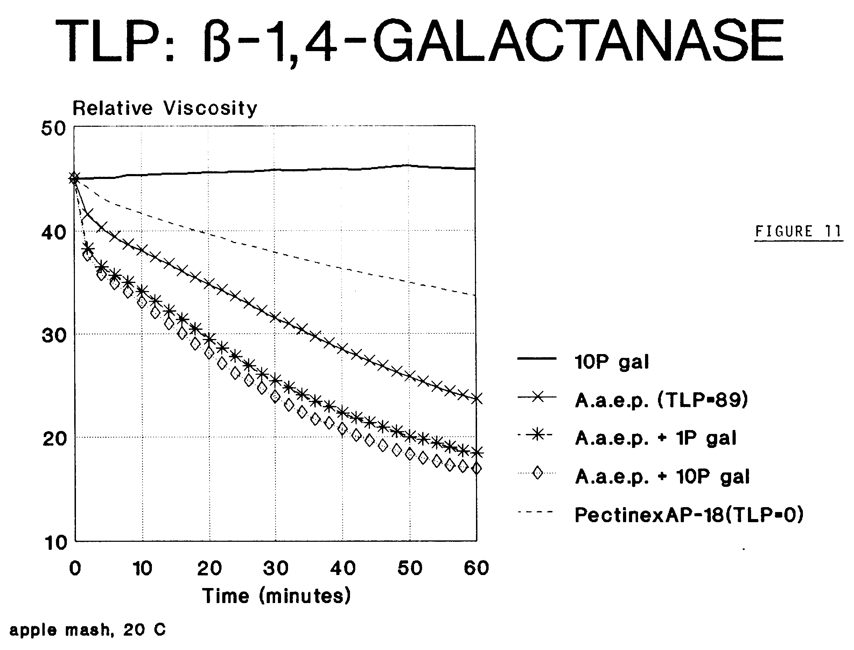

Also, reference is made to Fig. 11. Both Table 3 and Fig. 11 show the marked synergism of the $\beta$-1,4-galactanase and the A.a.e.p. Thus, galactanase alone shows at higher dosages no effect at all on the viscosity. In lower dosages even an increase in viscosity can be observed. But surprisingly, in combination with the A.a.e.p. a very significant synergistic effect can be observed. An increase of about 30% (260%) of the galactanase units alone in the A.a.e.p. results in a TLP-increase of 60% (100%). This proves, that the $\beta$-1,4-galactanase is a key activity for the liquefaction process of plant cell walls.

Table 3

Total liquefaction power (TLP) the A.a.e.p.: β-1,4-galactanase

| | Galactanase units | MOU/100 g mash | Protein: mg/ml Biorad | % protein added to A.a.e.p. protein Biorad | TLP expected % | TLP obtained % | TLP increase % |
|---|---|---|---|---|---|---|---|
| A.a.e.p. | 4.2 | 20 | 0.791 + 0 | 0 | 89 | 89 | 0 |
| A.a.e.p.+1P gal | 4.2+1.1 | 20+0.015 | 0.791+0.015 | 1.9 | 89 | 142 | 60 |
| A.a.e.p.+10P gal | 4.2+11 | 20+0.15 | 0.791+0.15 | 19 | 89 | 181 | 103 |
| 10P gal | 11 | 0.15 | 0.15 | - | ? | (-25) | - |

## Claims

1. β-1,4-galactanase with the following partial amino acid sequence, preferably the following N-terminal amino acid sequence

```
1                    5                      10
Ala-Leu-Thr-Tyr-Arg-Gly-Ala-Asp-Ile-Ser-Xaa-Leu-Leu-Leu

15                   20                     25
Leu-Glu-Asp-Glu-Gly-Tyr-Ser-Tyr-Lys-Asn-Leu-Asn-Gly-Gln-
```

or a partial amino acid sequence, preferably an N-terminal amino acid sequence with a homology thereto of at least 70%, preferably at least 80%, more preferably at least 90%.

2. $\beta$-1,4-galactanase according to Claim 1, obtainable by means of *Aspergillus aculeatus.*

3. A recombinant DNA sequence comprising a DNA sequence coding for a polypeptide having $\beta$-1,4-galactanase activity, or a DNA sequence having substantial sequence homology to such $\beta$-1,4-galactanase coding sequence.

4. A recombinant DNA sequence according to Claim 3, wherein the $\beta$-1,4-galactanase activity originates from the $\beta$-1,4-galactanase producible by means of *Aspergillus aculeatus* CBS 101.43 with the partial amino acid sequence according to Claim 1.

5. Vector comprising the recombinant DNA sequence according to Claim 3 or 4.

6. Vector according to Claim 5, wherein the promoter is the *Aspergillus oryzae* takaamylase promoter.

7. Transformed host containing the vector according to Claims 5 or 6.

8. Transformed host according to Claim 7, wherein the transformed host is an *Aspergillus* strain.

9. Transformed host according to Claim 8, wherein the transformed host is a strain belonging to the species *Aspergillus aculeatus, Aspergillus niger, Aspergillus oryzae* or *Aspergillus awamori.*

10. Transformed host according to Claim 7, wherein the transformed host is a microorganism, which in its non-transformed condition does not produce $\beta$-1,4-galactanase or only produces $\beta$-1,4-galactanase in insignificant amounts, preferably *Bacillus sp., E. coli* or *S. cerevisiae.*

11. Method for production of a $\beta$-1,4-galactanase by use of a transformed host according to Claims 7 - 10.

12. $\beta$-1,4-galactanase produced by the method according to Claim 11.

13. Enzyme preparation characterized by the fact that it contains a pectinase preparation usable for degradation or modification of plant cell walls enriched with an $\beta$-1,4-galactanase, preferably with an enrichment factor of at least 1.1 or deprived of an $\beta$-1,4-galactanase, preferably with a deprivation factor of maximum 0.9.

14. Enzyme preparation according to Claim 13, wherein the pectinase preparation is producible by means of a microorganism belonging to the genus *Aspergillus*, preferably *Aspergillus niger, Aspergillus aculeatus, Aspergillus awamori* or *Aspergillus oryzae*.

15. Enzyme preparation according to Claim 13 or 14, wherein the $\beta$-1,4-galactanase is the $\beta$-1,4-galactanase according to Claim 12.

16. Use of the $\beta$-1,4-galactanase as an agent for degradation or modification of plant cell walls.

17. Use according to Claim 16, wherein the $\beta$-1,4-galactanase is the $\beta$-1,4-galactanase according to Claim 12.

18. Use of the enzyme preparation according to Claims 13 - 15, as an agent for degradation or modification of plant cell walls.

FIG. 1

## A.a.e.p.: ß-1,4-galactanase
### Phenyl Sepharose CL4B

Fig. 2

## A.a.e.p.: ß-1,4-galactanase
### Accell DEAE

Fig. 3

## A.a.e.p.: ß-1,4-galactanase
### Protein PAK Phenyl 5PW

Fig. 4

## A.a.e.p.: ß-1,4-GALACTANASE
## pH Optimum

Fig. 5

Nelson Somogyi, 30 min
0.5% potato galactan (NNFAG)

## A.a.e.p.: ß-1,4-GALACTANASE
## pH Stability

Fig. 6

Nelson Somogyi, 30 min.
0.5% potato galactan (NNFAG)

## A.a.e.p.: ß-1,4-GALACTANASE
## Temperature Optimum

Fig. 7

**Relative Activity %**

Nelson Somogyi, 30 min.
0.5 % potato galactan (NNFAG)
0.05 M Na-Ac-buffer, pH4.5

## A.a.e.p.: ß-1,4-GALACTANASE
## Temperature Stability

Fig. 8

**Residual Activity %**

Nelson Somogyi (30 C, 30 min.)
0.5% galactan (NNFAG)
0.05m Na-Ac-buffer, pH 4.5

# A.a.e.p.: ß-1,4-GALACTANASE
## Michaelis Menten-Kinetic

Fig. 9

Velocity/0.5 min.

V max

1/2 V max

Km=0.412%

% Substrate

Nelson Somogyi, 30 min.
potato galactan (NNFAG)
0.05 M Na-Ac-buffer, pH 4.5

# A.a.e.p.: ß-1,4-GALACTANASE
## Lineweaver-Burk-Kinetic

Fig. 10

1/V

1/%S

Nelson Somogyi, 30 min.
0.5% potato galactan (NNFAG)
0.05 M Na-Ac-buffer pH 4.5

# TLP: ß-1,4-GALACTANASE

EP 0 498 137 A1

FIGURE 11

Relative Viscosity

apple mash, 20 C

Legend:
—— 10P gal
—×— A.a.e.p. (TLP=89)
-*- A.a.e.p. + 1P gal
--◇-- A.a.e.p. + 10P gal
- - - PectinexAP-18(TLP=0)

Time (minutes)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | FR-A-2518570 (NOVO INDUSTRI A/S.)<br>* page 9, line 26 - page 10, line 5; claims 1-7 *<br>* page 10, lines 29 - 28 * | 1, 3, 5,<br>7, 11,<br>13, 16 | C12N15/56<br>C12N9/24 |
| A | | 2 | |
| Y | Derwent Publications Ltd.,LONDON,GB; DATABASE WPIL,accession no. 90-372009,week 9050;<br>& JP-A-2268685[JOZO SHIGEN KENKYUS],02.11.1990 | 1, 3, 5,<br>7, 11 | |
| Y | EP-A-221811 (COMITE ECONOMIQUE AGRICOLE DE LA PRODUCTION DU CHANVRE ET AL.)<br>* abstract; claims 1-10 * | 13, 16 | |
| A | Derwent Publications Ltd.,LONDON,GB; DATABASE WPIL,accesssion no. 91-041066,week 9106;<br>& JP-A-2308798 [OSAKA YUKI KAGAKU K ET AL.]<br>21.12.90 | 1 | |
| A | Derwent Publications Ltd.,LONDON,GB; DATABASE WPIL,accesssion no. 90-018119,week 9003;<br>& JP-A-1296995 [OSAKA YUKI KAGAKU K ET AL.]<br>30.11.89 | 1 | TECHNICAL FIELDS SEARCHED (Int. Cl.5 )<br><br>C12N |

The present search report has been drawn up for all claims

| Place of search | Date of completion of the search | Examiner |
|---|---|---|
| BERLIN | 22 AUGUST 1991 | GURDJIAN D. |